# EUROPEAN PATENT APPLICATION

(11) **EP 4 226 871 A1**
(43) Date of publication of application: **16.08.2023**
(21) Application number: 22778379.2
(22) Date of filing: 15.02.2022
(51) Int. Cl.: A61B 17/064, B21G 3/00

(54) **MEDICAL ANCHOR AND PROCESSING TECHNOLOGY THEREFOR**

(30) Priority: 30.03.2021 CN 202110338258
(71) Applicant: Suzhou Origin Medical Technology Co., Ltd, Changshu, Jiangsu 215513 (CN)
(72) Inventor: ZHANG, Xiaonong, Suzhou, Jiangsu 215513 (CN); XU, Haidong, Suzhou, Jiangsu 215513 (CN); WANG, Xiao, Suzhou, Jiangsu 215513 (CN); CAO, Yingjia, Suzhou, Jiangsu 215513 (CN)
(74) Representative: Schmitt-Nilson Schraud Waibel Wohlfrom Patentanwälte Partnerschaft mbB
(86) International application number: PCT/CN2022/076273
(87) International publication number: WO 2022/206194

(57) **Abstract**

Disclosed is a medical anchor, including a head part (1), a body part (2) and a tail part (3) which are integrally processed. The head part (1) is conical. The body part (2) is smoothly connected to the head part (1). A screw thread (21) is processed on the body part (2). A wire-passing structure is designed on the tail part (3), and a connecting wire is assembled on the wire-passing structure of the tail part (3). The cross section of a tooth profile of the screw thread (21) is in the shape of a trapezoid, the ratio of an upper edge (22) to a lower edge (23) of the trapezoid on the cross section of the tooth profile of the screw thread (21) is 1:3-1:6, the trapezoidal cross section of the tooth profile of the screw thread (21) is a non-isosceles trapezoid, and the side of the trapezoid on the cross section of the tooth profile of the screw thread (21) having a shorter waist length is close to one side of the tail part. When the anchor uses a trapezoidal tooth profile, the tooth thickness is relatively large, and early-stage degradation of the pure magnesium anchor can be slowed down, thereby ensuring that the anchor can be firmly fixed in a bone in the early stage when the anchor is used in a human body. The recovery effect of the user is greatly improved.

## Description

### TECHNICAL FIELD

The present disclosure relates to the technical field of medical devices, and in particular to a medical anchor.

### BACKGROUND

People often encounter tendon or ligament injuries during exercise. When the tendons or ligaments are seriously injured, for example, cut by a sharp instrument or violent pulling, neat rupture, tendinous tissue avulsion with horsetail shaped section, avulsion fracture of the bone surface. Traditional medical repair methods are often used, such as plaster or bandage external fixation methods, suturing methods with steel wires, suturing methods with sutures, tendinous tissue fixation after bone opening, etc. However, these traditional methods are not firm in fixation, take a long time and have poor curative effect, and even lead to joint adhesion and stiffness, or even tissue rupture again, resulting in residual pain and even partial loss of function at the trauma site. In modern medicine, threaded anchors are often used to fix the tendon or ligament at the strain position. In order to reduce the hole where the anchor is installed on the bone, the size of anchor is usually reduced as much as possible, but the reduction of the size of the anchor will make the overall hardness and tensile strength of the anchor. In addition, the anchor used in medical operation at present is made of a degradable material. Due to the small size and rapid degradation rate of the anchor, the anchor often fall out before the surgical sutured or fixed tendon or ligament is fully grown, which greatly affects the outcome of the surgery. Screw thread is processed on the anchor to grip the anchor in the bone tightly. The traditional anchor is sharp in the tooth profile of the screw thread and is easy to degrade quickly in early stage of use, which reduces the grip between the anchor and the bone and affects the use effect of the anchor.

### SUMMARY

For the problem above, an objective of the present disclosure is to provide a medical anchor which has a simple structure, and can guarantee the strength of the anchor while slowing down early-stage degradation of the anchor.

A technical solution adopted by the present disclosure for solving the technical problem is as follows: a medical anchor includes a head part, a body part and a tail part which are integrally processed. The head part is conical. The body part is smoothly connected to the head part. A screw thread is processed on the body part. A wire-passing structure is designed on the tail part, and a connecting wire is assembled on the wire-passing structure of the tail part. The cross section of a tooth profile of the screw thread is in the shape of a trapezoid, the ratio of an upper edge to a lower edge of the trapezoid on the cross section of the tooth profile of the screw thread is 1 :3-1:6, the trapezoidal cross section of the tooth profile of the screw thread is a non-isosceles trapezoid, and the side of the trapezoid on the cross section of the tooth profile of the screw thread having a shorter waist length is close to one side of the tail part.

As a preference, the anchor is made of a pure magnesium material with a purity of 99.99%.

As a preference, a ratio of an upper edge to a lower edge of the trapezoid on the cross section of the tooth profile of the screw thread is 1:4-1:5.

As a preference, the body part in the shape of a cylinder. A height of the tooth profile of the screw thread at a connection position of the body part and the head part gradually increases with a spiral path.

As a preference, the body part prior to processing is in the shape of a cylinder, and the screw thread is processed on the body part by cutting.

As a preference, the cross section of the tail part is hexagonal, wire passage grooves are processed on left and right symmetrical surfaces, respectively, and a through wire-passing hole is processed on the left and right symmetrical wire passage grooves. Each of the wire passage grooves is a rectangular groove and is parallel to the axis of the anchor, and the cross section of the wire passage groove is rectangular.

A processing technology for the medical anchor includes the following steps:
(1) taking a pure magnesium rod with a diameter of 7.0 mm to 12.0 mm and a purity of greater than 99.9% as a raw material, and cutting the magnesium rod into a set size;
(2) carrying out cold rolling on the magnesium rod in step 1 for more than two passes, and cold-rolling a diameter of the magnesium rod to 4.0-6.0 mm;
(3) processing the magnesium rod after cold rolling in step 2 into the shape of an anchor;
(4) carrying out electrochemical polishing on the anchor in step 3 through a phosphoric acid-ethylene glycol system;
(5) applying a magnesium fluoride coating to the outside of the polished anchor in step 4; and
(6) collecting and storing the coated anchor in step 5.

As a preference, the magnesium rod material in step 1 has a diameter from 8.0 mm to 10.0 mm. The magnesium rod after three to eight passes of cold rolling in step 2 has a dimeter from 4.0 mm to 5.0 mm.

As a preference, the purity of the magnesium rod material in step 1 is 99.99%.

As a preference, the magnesium fluoride coating in step 5 is replaced with a polylactic acid coating.

As a preference, one step is added between step 5 and step 6, including further applying a polylactic acid coating to the outside of the magnesium fluoride coating on the anchor.

The present disclosure has the beneficial effects that the medical anchor is mainly used to fix tendon or ligament on a bone when the tendon or ligament is torn or strained. The ligament or tendon is pulled to its original position by a pull wire at the tail part, thus making the ligament or tendon recovered to its normal position for growing. After the growth of tendon or ligament is completed, the anchor and connecting wire are degraded in the body, thus making the ligament and tendon completely recovered to their original state. A tooth profile used by the anchor is a tooth profile with a trapezoidal cross section, and a degradation rate at a sharp position is faster according to the degradation characteristics of pure magnesium. Therefore, when the anchor uses a trapezoidal tooth profile, the tooth thickness is relatively large, and early-stage degradation of the pure magnesium anchor can be slowed down, thereby ensuring that the anchor can be firmly fixed in a bone in the early stage when the anchor is used in a human body. The recovery effect of the user is greatly improved.

### BRIEF DESCRIPTION OF THE DRAWINGS

FIG. 1 is a schematic diagram of a three-dimensional structure of an anchor in accordance with the present disclosure.
FIG. 2 is a schematic diagram of a frontal structure of an anchor in accordance with the present disclosure.
FIG. 3 is a structural schematic diagram of an anchor in accordance with the present disclosure directly facing a tail direction.
FIG. 4 is a cross-section diagram of a tooth profile of a screw thread of a traditional anchor.
FIG. 5 is a cross-section diagram of a tooth profile of a screw thread of an anchor in accordance with the present disclosure.
FIG. 6 is a cross-section diagram of a tail part of a traditional anchor.
FIG. 7 is a cross-section diagram of a tail part of an anchor in accordance with the present disclosure.

### DETAILED DESCRIPTION OF THE EMBODIMENTS

The present disclosure is further described below with reference to accompanying drawings and embodiments.

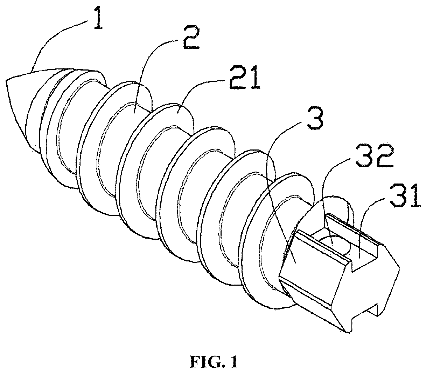

As shown in embodiments in FIG. 1, FIG. 2 and FIG. 3, similar to a traditional anchor structure, a medical anchor provided by the present disclosure includes a head part 1, a body part 2 and a tail part 3 which are integrally processed. The head part 1 is conical. The body part 2 is smoothly connected to the head part 1. A screw thread 21 is processed on the body part 2. A wire-passing structure is designed on the tail part 3, and a connecting wire is assembled on the wire-passing structure of the tail part 3. The features of the anchor according to the present disclosure are that the cross section of a tooth profile of the screw thread 21 is in the shape of a trapezoid, a ratio of an upper edge 22 to a lower edge 23 of the trapezoid on the cross section of the tooth profile of the screw thread 21 is 1:3-1:6, the trapezoidal cross section of the tooth profile of the screw thread 21 is a non-isosceles trapezoid, and the side of the trapezoid on the cross section of the tooth profile of the screw thread 21 having a shorter waist length is close to one side of the tail part. That is, the tooth profile of the screw thread 21 is integrally inclined towards the tail part. A force on the anchor is mainly from the tension of the connecting wire on the tail part 3 when connecting tendon or ligament. The inclined design of the tooth profile makes the tooth profile of the anchor have smaller slope in a force direction and thus have stronger grip.

The medical anchor is mainly used to fix tendon or ligament on a bone when the tendon or ligament is torn or strained. The ligament or tendon is pulled to its original position by a pull wire at the tail part, thus making the ligament or tendon recovered to its normal position for growing. After the growth of tendon or ligament is completed, the anchor and connecting wire are degraded in the body, thus making the ligament and tendon completely recovered to their original state. A tooth profile used by the screw thread 21 of the anchor is a tooth profile with a trapezoidal cross section, and a degradation rate at a sharp position is faster according to the degradation characteristics of pure magnesium. Therefore, when the anchor uses a trapezoidal tooth profile, the tooth thickness is relatively large, and early-stage degradation of the pure magnesium anchor can be slowed down, thereby ensuring that the anchor can be firmly fixed in a bone in the early stage when the anchor is used in a human body. The recovery effect of the user is greatly improved.

During specific design, the anchor is made of a pure magnesium material with a purity of 99.99%. A high-purity magnesium rod, which is used as the raw material, makes the composition of the material simple and the degradation product simple, which is beneficial to the safety of the degradation product.

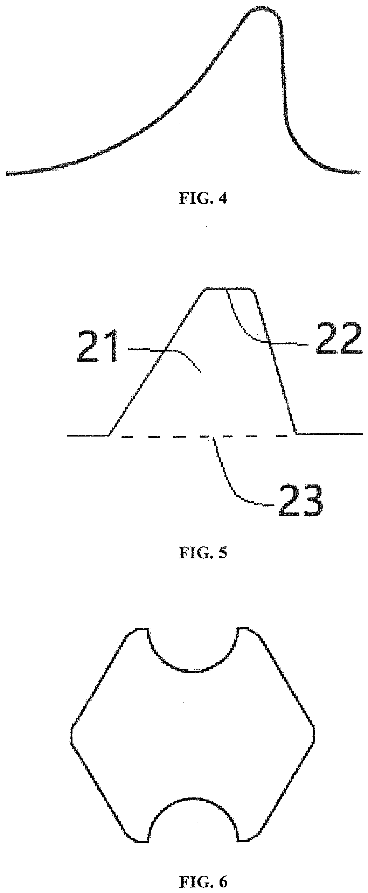

A tooth profile of a screw thread of a traditional anchor is as shown in FIG. 4, a head part of the tooth profile is relatively sharp. During specific design in this embodiment, a ratio of an upper edge 22 to a lower edge 23 of the trapezoid on the cross section of the tooth profile of the screw thread 21 is 1:4-1:5. Such a size has the optimal performance, and can guarantee the grip of the anchor while guaranteeing the strength of the anchor to the greatest extent. Meanwhile, a pitch between the screw threads is greater than 2 times the width of the lower edge 23 at the bottom of the screw thread 21, and thus there is more width between the screw threads for the anchor to grip the bone firmly.

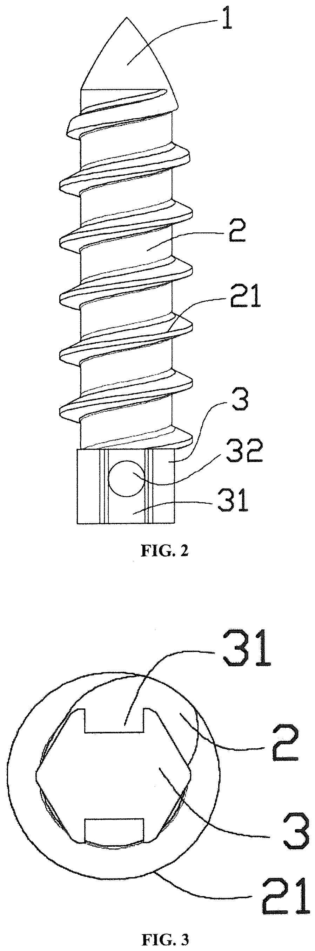

As shown in FIG. 1 and FIG. 2, the body part 2 in the shape of a cylinder, a height of the tooth profile of the screw thread 21 at a connection position of the body part 2 and the head part 1 gradually increases with a spiral path. In particular, the body part 2 prior to processing is in the shape of cylinder, and the head part 1 is also in the shape of a cone and is smoothly connected to the body part 2. A base material is subjected to cutting so as to cut screw threads 21 on the body part. Therefore, the conical shape of the head part 1 and part of the screw threads 21 retain the original conical structure, and the whole anchor can be driven into a bone more smoothly. Meanwhile, such a processing mode has less technological processes, high processing speed and low processing cost.

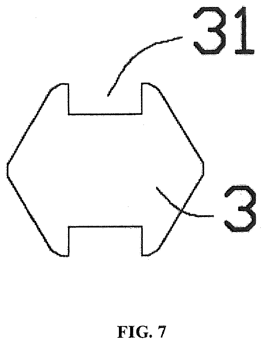

The anchor needs to be screwed in a bone mass with a special medical operation tool when used, and thus a hexagonal prism structure is used at the tail part to be in transmission connection with an operated medical device. A tail part of the traditional anchor is as shown in FIG. 6, the cross section of the tail part is hexagonal, and a structure with symmetric grooves is used. The cross section of the groove is a semi-circular groove structure, and the thickness of such a groove structure is narrow between the semi-circular grooves of the cross section, and thus when the tail part is subjected to torsion, the bearing capacity of this structure is low. In this embodiment, as shown in FIG. 1, FIG. 2, FIG. 3 and FIG. 7, a tail part 3 with a hexagonal cross section is also used, and wire passage grooves 31 are respectively processed on left and right symmetrical surfaces of the tail part 3, and a wire-passing hole 32 is processed on the left and right symmetrical wire passage grooves 31, and the connecting wire is connected into the wire-passing hole. In this embodiment, each of the wire passage grooves 31 is a rectangular groove and is parallel to the axis of the anchor, and the cross section of the wire passage groove 31 is rectangular. Under the same wire passage volume, such a wire passage groove 31 structure makes the distance between bottoms of two wire passage grooves 31 larger than the tail part structure of the traditional anchor, and the wire passage groove 31 structure has higher torsional strength. Meanwhile, the hollow wire-passing hole 32 has remarkable structural advantages: a suture can pass through the inside of the anchor, and the pull-out resistance is better.

A processing technology for the medical anchor includes the following steps:
(1) A pure-magnesium rod with a diameter of 7.0 mm to 12.0 mm and a purity of greater than 99.9% is used as a raw material, and the magnesium rod is cut into a set size.
(2) The magnesium rod in step one is subjected to more than two passes of cold rolling, and a diameter of the magnesium rod is cold-rolled to 4.0-6.0 mm.
(3) The magnesium rod after cold rolling in step 2 is processed into the shape of an anchor.
(4) The anchor in step 3 is subjected to electrochemical polishing through a phosphoric acid-ethylene glycol system.
(5) A magnesium fluoride coating is applied to the outside of the polished anchor in step 4.
(6) The coated anchor in step 5 is collected and stored.

Compared with the traditional processing technology, the processing technology for the medical anchor implements new technological flows to make the anchor meet new medical demands. After the processed anchor is subjected to cold rolling in step 2, the hardness of the magnesium rod is improved by 10% to 20%, and the tensile strength is improved by 20% to 30%. Therefore, the finally processed anchor has higher hardness and strength under the same size specification, and the anchor can be driven into a bone of a patient more firmly, is not easy to be damaged by a force, and has better operation effect. After the anchor in step 3 is subjected to electrochemical polishing through the phosphoric acid-ethylene glycol system, the surface of the anchor obtains a bright protective layer with antioxidant capacity, which can slow down the degradation rate in the early stage of implantation and ensure the mechanical properties in the early stage of implantation. High-purity magnesium rod, which is used as the raw material, makes the composition of the material simple and the degradation product simple, which is beneficial to the safety of the degradation product. Moreover, the magnesium fluoride coating in step 5 is a degradable coating, with a main function of slowing down the degradation rate in the early stage of implantation and ensuring the mechanical properties.

In addition, by using the high-purity magnesium material as a base material of the anchor, the anchor can be quickly absorbed by a human body after the outer protective layer and coating are consumed, which not only can ensure the structural stability of the anchor in the early stage of use in the human body, but also facilitates the recovery of tendons or ligaments. Meanwhile, the anchor can be quickly absorbed after the outer protective layer and coating are consumed in the later stage of use, thus preventing the existence of the anchor from affecting the internal structure of the human body. The use effect of the anchor is greatly improved. In this way, the use of the anchor can greatly reduce the whole treatment time under the condition of ensuring the treatment effect, and the treated person can return to normal more quickly.

During specific implementation, the magnesium rod material used in step 1 has a diameter from 8.0 mm to 10.0 mm. The magnesium rod in step 2 is subjected to cold rolling until the diameter is from 4.0 mm to 5.0 mm. The magnesium rod in step 1 needs to be cold-rolled for three to eight passes. 4.0-5.0 mm is convenient for the processing of the size of the anchor. Excessive cold rolling for the magnesium rod is easy to damage the magnesium rod, while less cold rolling for the magnesium rod cannot meet the requirements of improving mechanical properties.

During specific use, the purity of the magnesium rod material in step 1 is 99.99%. The magnesium rod with higher purity guarantees simple composition of the material, makes the degradation product simpler, and greatly improves the safety of the degradation product.

During specific operation, the magnesium fluoride coating in step 5 can be replaced with a polylactic acid coating. Alternatively, one step can be added between step 5 and step 6, including further applying a polylactic acid coating to the outside of the magnesium fluoride coating on the anchor. The dual-coating structure further improves the consumption time of the coating when the anchor is used in the human body, and the tendon or ligament has a longer recovery time when the anchor is used to treat tendon or ligament injuries.

The above is only the preferred mode of the disclosure and is not used to limit the present disclosure. Any modification, equivalent substitution, improvement, etc. made within the spirit and principle of the present disclosure should be included in the scope of protection of the present disclosure.

## Claims

1. A medical anchor, comprising a head part (1), a body part (2) and a tail part (3) which are integrally processed, wherein the head part (1) is conical, the body part (2) is smoothly connected to the head part (1), a screw thread (21) is processed on the body part (2), a wire-passing structure is designed on the tail part (3), and a connecting wire is assembled on the wire-passing structure of the tail part (3); the cross section of a tooth profile of the screw thread (21) is in the shape of a trapezoid, a ratio of an upper edge (22) to a lower edge (23) of the trapezoid on the cross section of the tooth profile of the screw thread (21) is 1:3-1:6, the trapezoidal cross section of the tooth profile of the screw thread (21) is a non-isosceles trapezoid, and the side of the trapezoid on the cross section of the tooth profile of the screw thread (21) having a shorter waist length is close to one side of the tail part.

2. The medical anchor according to claim 1, wherein the anchor is made of a pure magnesium material with a purity of 99.99%.

3. The medical anchor according to claim 1, wherein a ratio of an upper edge (22) to a lower edge (23) of the trapezoid on the cross section of the tooth profile of the screw thread (21) is 1:4-1:5.

4. The medical anchor according to claim 1, wherein the body part (2) in the shape of a cylinder, a height of the tooth profile of the screw thread (21) at a connection position of the body part (2) and the head part (1) gradually increases with a spiral path, and the screw thread (21) is processed on the body part (2) by cutting.

5. The medical anchor according to claim 1, wherein the cross section of the tail part (3) is hexagonal, wire passage grooves (31) are processed on left and right symmetrical surfaces, respectively, and a through wire-passing hole (32) is processed on the left and right symmetrical wire passage grooves (31); each of the wire passage grooves (31) is a rectangular groove and is parallel to the axis of the anchor, and the cross section of the wire passage groove (1) is rectangular.

6. A processing technology for the medical anchor according to any one of claims 1 to 5, comprising the following steps:
(1) taking a pure magnesium rod with a diameter of 7.0 mm to 12.0 mm and a purity of greater than 99.9% as a raw material, and cutting the magnesium rod into a set size;
(2) carrying out cold rolling on the magnesium rod in step 1 for more than two passes, and cold-rolling a diameter of the magnesium rod to 4.0-6.0 mm;
(3) processing the magnesium rod after cold rolling in step 2 into the shape of an anchor;
(4) carrying out electrochemical polishing on the anchor in step 3 through a phosphoric acid-ethylene glycol system;
(5) applying a magnesium fluoride coating to the outside of the polished anchor in step 4; and
(6) collecting and storing the coated anchor in step 5.

7. The processing technology for the medical anchor according to claim 6, wherein the magnesium rod material in step 1 has a diameter from 8.0 mm to 10.0 mm, and the magnesium rod after three to eight passes of cold rolling in step 2 has a dimeter from 4.0 mm to 5.0 mm.

8. The processing technology for the medical anchor according to claim 6, wherein the magnesium fluoride coating in step 5 is replaced with a polylactic acid coating.

9. The processing technology for the medical anchor according to claim 6, wherein one step is added between step 5 and step 6, comprising further applying a polylactic acid coating to the outside of the magnesium fluoride coating on the anchor.
